# EUROPÄISCHE PATENTSCHRIFT

(11) **EP 2 903 955 B1**
(45) Veröffentlichungstag und Bekanntmachung des Hinweises auf die Patenterteilung: **18.01.2017**
(21) Anmeldenummer: 13767004.8
(22) Anmeldetag: 30.09.2013
(51) Int. Cl.: C07C 7/08

(54) **VERFAHREN ZUR AUFTRENNUNG EINES GEMISCHES VON KOHLENWASSERSTOFFEN DURCH EXTRAKTIVDESTILLATION**
METHOD FOR SEPARATING A MIXTURE OF HYDROCARBONS BY EXTRACTIVE DISTILLATION
PROCÉDÉ DE SÉPARATION D'UN MÉLANGE D'HYDROCARBURES PAR DISTILLATION EXTRACTIVE

(30) Priorität: 01.10.2012 EP 12186826
(43) Veröffentlichungstag der Anmeldung: 12.08.2015
(73) Patentinhaber: BASF SE, 67056 Ludwigshafen am Rhein (DE)
(72) Erfinder: BRAND, Raphael Heinrich, 67273 Weisenheim am Berg (DE); ASPRION, Norbert, 67063 Ludwigshafen (DE); FERSTL, Wolfgang, 76135 Karlsruhe (DE); TELLAECHE HERRANZ, Carlos, 69126 Heidelberg (DE); GÖTZ, Jochen, 67346 Speyer (DE); REZAI, Alireza, 68163 Mannheim (DE)
(74) Vertreter: BASF IP Association
(86) Internationale Anmeldenummer: PCT/EP2013/070321
(87) Internationale Veröffentlichungsnummer: WO 2014/053431

(56) Entgegenhaltungen:
- DE-A1- 10 219 375

## Beschreibung

Gegenstand der Erfindung ist ein Verfahren zur Auftrennung eines Gemisches von Kohlenwasserstoffen durch Extraktivdestillation mit einem Gemisch aus einem polaren aprotischen Lösungsmittel und Wasser als selektivem Lösungsmittel.

Extraktivdestillationen werden in der Regel für komplexe destillationstechnische Trennaufgaben eingesetzt, insbesondere wenn sich die relativen Flüchtigkeiten der aufzutrennenden Komponenten nur geringfügig unterscheiden, und somit eine klassische destillative Auftrennung nicht oder nur mit einem hohen Aufwand möglich ist, werden häufig so genannte Extraktivdestillationen eingesetzt, das heißt Destillationen unter Zugabe eines selektiven Lösungsmittels (auch als Extraktionsmittel bezeichnet), das einen höheren Siedepunkt als das aufzutrennende Gemisch aufweist und das die Unterschiede in den relativen Flüchtigkeiten der aufzutrennenden Komponenten erhöht.

Häufig werden als selektive Lösungsmittel Gemische aus polaren aprotischen Lösungsmitteln mit Wasser eingesetzt. Deren Selektivität ändert sich mit ihrem Wassergehalt. Entsprechend ändert sich mit dem Wassergehalt des selektiven Lösungsmittels auch die Zusammensetzung der in der Extraktivdestillation erhaltenen Produktströme.

Die weiter verarbeitenden Betriebe erwarten jedoch eine konstante Produktqualität, bzw. werden Schwankungen der Produktzusammensetzung nur in sehr geringen Grenzen toleriert.

Extraktivdestillationen werden in der Regel in der Weise betrieben, dass sich das selektive Lösungsmittel in einer Extraktivdestillationskolonne mit den Komponenten aus einem aufzutrennenden Gemisch von Kohlenwasserstoffen anreichert, für die es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck gegenüber dem ersten Verfahrensschritt in einem oder mehreren weiteren Verfahrensschritten die Komponenten fraktioniert aus dem selektiven Lösungsmittel in einer Ausgaserkolonne freigesetzt, unter Erhalt eines gereinigten Lösungsmittels, das erneut in die Extraktivdestillationskolonne recycliert wird. Da Wasser jedoch gegenüber den polaren aprotischen Lösungsmitteln ein Leichtsieder ist, wird es in der Ausgaserkolonne aus dem Lösungsmittel ausgeschleppt, so dass bei einer kontinuierlichen Verfahrensführung der Anteil desselben im recyclierten Lösungsmittelstrom abnimmt.

Zu Schwankungen in der Wasserkonzentration des selektiven Lösungsmittels kann es auch dadurch kommen, dass der in der Extraktivdestillation eingesetzte Feedstrom von Kohlenwasserstoffen noch Wasser in unterschiedlicher Konzentration enthält, beispielsweise, wenn in der Extraktivdestillation ein C₄-Kohlenwasserstoffe enthaltender Strom eingesetzt wird, der aus einer Butandehydrierung stammt, und woraus das in der Butandehydrierung anfallende Wasser nicht vollständig kondensiert wurde.

Die Messung der Wasserkonzentration des selektiven Lösungsmittels erfolgte bislang in der Regel durch Karl-Fischer-Titration, einer oxidimetrischen Titration mit dem sogenannten Karl-Fischer- Reagenz, einer Maßlösung, die Jod (als Oxidationsmittel) Schwefeldioxid (als Reduktionsmittel), Pyridin und wasserfreies Methanol enthält. Die Wirkungsweise des Reagenzes beruht darauf, dass der Redoxvorgang durch die noch fehlende Komponente des Redoxsystems, das in der zu untersuchenden Probe vorhandene Wasser, ausgelöst wird. Der Titrationsendpunkt gibt sich durch einen Jod-Überschuss zu erkennen, der visuell, photometrisch oder elektrometrisch angezeigt werden kann.

Der Titrationsendpunkt wird jedoch durch die Anwesenheit von Basen, insbesondere auch durch polare aprotische Lösungsmittel mit elektronreichen Heteroatomen, wie Schwefel, Stickstoff oder Sauerstoff verändert.

Es hat sich gezeigt, dass eine Wiederverwendung des Karl-Fischer-Reagenzes bereits nach dem ersten Messeinsatz nicht mehr zu zuverlässigen Messergebnissen führt, wobei angenommen wird, dass die Basizität des polaren aprotischen Lösungsmittels ursächlich für die Verfälschung des Titrationsendpunktes ist.

Es war demgegenüber Aufgabe der Erfindung, ein Verfahren zur Verfügung zu stellen, das eine konstante Produktqualität gewährleistet und das kontinuierlich, bevorzugt automatisch, betrieben werden kann.

Die Aufgabe wird gelöst durch ein kontinuierliches Verfahren zur Auftrennung eines Gemisches von Kohlenwasserstoffen durch Extraktivdestillation mit einem Gemisch aus einem polaren aprotischen Lösungsmittel und Wasser als selektivem Lösungsmittel, in einer Anlage umfassend eine Extraktivdestillationskolonne und eine Ausgaserkolonne, die dadurch gekennzeichnet ist, dass das Verfahren unter kontinuierlicher Messung des Wassergehaltes des selektiven Lösungsmittels durch NIR-Spektroskopie durchgeführt wird, wobei die Messwerte aus der kontinuierlichen Messung des Wassergehaltes des selektiven Lösungsmittels mit einem vorgegebenen Soll-Wert verglichen und durch Ergänzung oder Ausschleusung von Wasser oder durch Ergänzung von polarem aprotischen Lösungsmittel auf einen Wert innerhalb eines vorgegebenen Konzentrationsbereichs oberhalb und unterhalb des Sollwerts geregelt werden.

Das vorliegende Verfahren kann für jedes Gemisch von Kohlenwasserstoffen, das durch Extraktivdestillation mit einem Gemisch aus einem polaren aprotischen Lösungsmittel und Wasser als selektivem Lösungsmittel aufgetrennt wird, eingesetzt werden.

Bevorzugt kann es sich bei dem Gemisch von Kohlenwasserstoffen um einen sogenannten C₄-Schnitt handeln, das heißt um ein Gemisch von Kohlenwasserstoffen mit überwiegend vier Kohlenstoffatomen pro Molekül.

C₄-Schnitte werden beispielsweise bei der Herstellung von Ethylen und/oder Propylen durch thermisches Spalten, üblicherweise in Steamcrackern oder FCC-Crackern (Fluidized Catalytic Cracking) einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an sonstigen Kohlenwasserstoffen, darunter Butine, insbesondere 1-Butin (Ethylacetylen) und Butenin (Vinylacetylen). Dabei beträgt der 1,3-Butadiengehalt von C₄-Schnitten aus Steamcrackern im Allgemeinen 10 bis 80 Gew.-%, vorzugsweise 20 bis 70 Gew.-%, insbesondere 30 bis 60 Gew.-%, während der Gehalt an Vinylacetylen und Ethylacetylen im Allgemeinen 5 Gew.-% nicht übersteigt.

Die Auftrennung von C₄-Schnitten ist wegen der geringen Unterschiede in den relativen Flüchtigkeiten der Komponenten ein kompliziertes destillationstechnisches Problem. Daher wird die Auftrennung in der Regel durch Extraktivdestillation durchgeführt.

Es ist eine Vielzahl von Verfahren zur Auftrennung von C₄-Schnitten mittels Extraktivdestillation unter Verwendung von selektiven Lösungsmitteln bekannt. Ihnen ist gemeinsam, dass sich durch Gegenstromführung des aufzutrennenden C₄-Schnittes in Dampfform mit dem flüssigen selektiven Lösungsmittel bei geeigneten thermodynamischen Bedingungen, in der Regel bei niedrigen Temperaturen, häufig im Bereich von 20 bis 80°C und bei moderaten Drücken, häufig bei Normaldruck bis 6 bar, das selektive Lösungsmittel mit den Komponenten aus dem C₄-Schnitt belädt, zu denen es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck gegenüber dem ersten Verfahrensschritt in einem oder mehreren weiteren Verfahrensschritten die Komponenten fraktioniert aus dem selektiven Lösungsmittel in einer Ausgaserkolonne freigesetzt.

Häufig wird die Extraktivdestillation von C₄-Schnitten in der Weise gefahren, dass die Komponenten des C₄-Schnittes, für die das selektive Lösungsmittel eine geringere Affinität als für 1,3-Butadien hat, insbesondere die Butane und die Butene, im Wesentlichen in der Gasphase verbleiben, wogegen 1,3-Butadien sowie weitere Kohlenwasserstoffe, für die das selektive Lösungsmittel eine höhere Affinität als für 1,3-Butadien hat, vom selektiven Lösungsmittel im Wesentlichen vollständig absorbiert werden. Die Gasphase wird als Kopfstrom abgezogen und häufig als Raffinat 1 bezeichnet. Ein derartiges Verfahren ist beispielsweise in der DE-A 198 188 10 beschrieben, wobei das Raffinat 1 der in den Figuren 1 und 2 mit Gbc bezeichnete Kopfstrom der Extraktivdestillationskolonne E I ist.

Für die weitere Verwertung des Raffinats 1 ist es jedoch in der Regel wirtschaftlicher, wenn die Butane und die Butene jeweils als getrennte Ströme vorliegen. Die in den nachfolgenden Stufen für die Weiterverarbeitung der Butene eingesetzten Apparate werden dadurch kleiner und Butane können direkt als wertvoller Cracker-Feed gewonnen werden.

Daher schlägt die DE-A 102 193 75 ein Verfahren zur Auftrennung eines C₄-Schnittes durch Extraktivdestillation vor, wonach Butane und Butene in jeweils getrennten Strömen erhältlich sind. Hierfür sind jedoch zwei Verfahrensstufen erforderlich, wobei in einer ersten Verfahrensstufe I in einer Waschzone ein die Butane enthaltender Kopfstrom und in einer zweiten Verfahrensstufe II in einer Ausgaserzone ein die Butene enthaltender Kopfstrom abgezogen wird.

Die Butane/Butene-Trennung kann vorteilhaft in einer einzigen Kolonne, die als Trennwandkolonne, mit bis zum oberen Ende derselben durchgezogener Trennwand, ausgebildet ist, durchgeführt werden, wie in der DE 102 33 620.2 beschrieben.

Weiter bevorzugt kann das Verfahren zur Auftrennung eines Gemisches von Kohlenwasserstoffen durch Extraktivdestillation mit einem Gemisch aus einem polaren aprotischen Lösungsmittel und Wasser als selektivem Lösungsmittel eine Extraktivdestillation eines C₄-Schnittes zu 1,3-Butedien als Wertprodukt, sein.

Der C₄-Schnitt kann bevorzugt durch Oxidehydrierung von Butenen gewonnen werden.

Für das vorliegende kontinuierliche Verfahren der Extraktivdestillation sind als polare aprotische Lösungsmittel Substanzen geeignet, deren Affinität ausgehend von Kohlenwasserstoffen mit Einfachbindungen in Richtung zu Kohlenwasserstoffen mit Doppelbindungen und weiter von Kohlenwasserstoffen mit konjugierten Doppelbindungen und Dreifachbindungen zunimmt. Aus appartetechnischen Gründen werden daher wenig oder nicht korrosive Substanzen bevorzugt.

Geeignete polare aprotische Lösungsmittel für das erfindungsgemäße Verfahren sind zum Beispiel Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon, im Folgenden abgekürzt als NMP bezeichnet. Im Allgemeinen werden alkylsubstituierte niedere aliphatische Säureamide oder N-alkylsubstituierte cyclische Säureamide verwendet. Besonders vorteilhaft sind Dimethylformamid, Dimethylsulfoxid, und insbesondere NMP.

Im erfindungsgemäßen Verfahren wird das polare aprotische Lösungsmittel als Gemisch mit Wasser als selektives Lösungsmittel eingesetzt.

Bevorzugt wird als selektives Lösungsmittel eine Lösung aus NMP und 7 bis 10 Gew.-% Wasser, besonders bevorzugt eine Lösung aus NMP und 8,3 Gew.-% Wasser, jeweils bezogen auf das Gesamtgewicht des selektiven Lösungsmittels, eingesetzt.

Erfindungsgemäß wird die kontinuierliche Messung des Wassergehaltes des selektiven Lösungsmittels durch NIR-Spektroskopie durchgeführt. Hierfür kann jedes handelsübliche NIR-Spektrophotometer eingesetzt werden. Das NIR-Spektrophotometer sollte vorteilhaft mit einer Messzelle ausgestattet sein, die auf den maximal zulässigen Anlagendruck ausgelegt ist, z.B. auf 15 bar absolut, oder auch auf 12 bar absolut.

Die durch NIR-Messung ermittelten Messdaten für den Wassergehalt können kontinuierlich im Prozessleitsystem angezeigt und kontinuierlich dokumentiert werden. Durch NIR-Messung ist der Wassergehalt kontinuierlich problemlos regelbar, insbesondere auf eine Genauigkeit von ± 0,5 %.

Bevorzugt können die Messwerte aus der kontinuierlichen Messung des Wassergehaltes des selektiven Lösungsmittels zur automatischen Regelung des Wassergehaltes des selektiven Lösungsmittels genutzt werden, indem bei Unterschreiten eines Mindestwertes eine Dosierpumpe in Betrieb gesetzt wird, die Wasser ergänzt oder bei Überschreiten eines Höchstwertes über ein Ventil am Rücklauf der Ausgaserkolonne Wasser ausgeschleust oder über eine Dosierpumpe polares aprotisches Lösungsmittel ergänzt wird.

Weiter bevorzugt können die Messwerte aus der kontinuierlichen Messung des Wassergehaltes des selektiven Lösungsmittels zur automatischen Regelung des Wassergehaltes des selektiven Lösungsmittels genutzt werden, indem bei Unterschreiten eines Mindestwertes bei zwei oder mehreren unmittelbar aufeinanderfolgenden Messungen eine Dosierpumpe in Betrieb gesetzt wird, die Wasser ergänzt oder bei Überschreiten eines Höchstwertes bei zwei oder mehreren unmittelbar aufeinanderfolgenden Messungen über ein Ventil am Rücklauf der Ausgaserkolonne Wasser ausgeschleust oder über eine Dosierpumpe polares aprotisches Lösungsmittel ergänzt wird.

Die Verwendung von zwei oder mehreren aufeinanderfolgenden Messwerten hat den Vorteil, dass ein Auslösen der Zudosierung bzw. Ausschleusung auf Grund eines Messausreissers ausgeschlossen wird. Die Dosier-bzw. Ausschleuszeit ist von der Dosier-bzw. Ausschleusrate, dem Lösungsmittel-Hold-up und den gewählten Grenzen der Unter- bzw. Überschreitung abhängig. Daraufhin erfolgt eine Wartezeit, bis sich die Pumpe wieder einschalten kann, um eine gute Durchmischung und damit repräsentative Messungen zu gewährleisten. Die Wartezeit richtet sich nach Volumenstrom und Lösungsmittel-Hold-up und liegt bevorzugt im Bereich von 1 bis 7 Verweilzeiten des Lösungsmittels im Lösungsmittelkreislauf.

Besonders bevorzugt ist ein Verfahren, das dadurch gekennzeichnet ist, dass die kontinuierliche Messung des Wassergehaltes des selektiven Lösungsmittel durch NIR-Spektroskopie an einer Stelle im Verfahren durchgeführt wird, an der das selektive Lösungsmittel von Kohlenwasserstoffen ausgegast ist.

Die Erfindung wird im Folgenden anhand einer Zeichnung sowie von Ausführungsbeispielen näher erläutert.

In der Zeichnung zeigen im Einzelnen:
Figur 1 eine Darstellung der Messwerte für die Wasserkonzentration des selektiven Lösungsmittels in einer Miniplantanlage zur Extraktivdestillation eines C4-Schnittes,
Figur 2 eine Darstellung der Messergebnisse mittels Karl-Fischer-Titration bei mehrmaliger Bestimmung des Wassergehaltes derselben Probe und
Figur 3 eine schematische Darstellung der Abhängigkeit des Gehaltes an Butenen in Raffinat 2 bzw. in einem Butane enthaltenden Produktstrom vom Wassergehalt des selektiven Lösungsmittels.

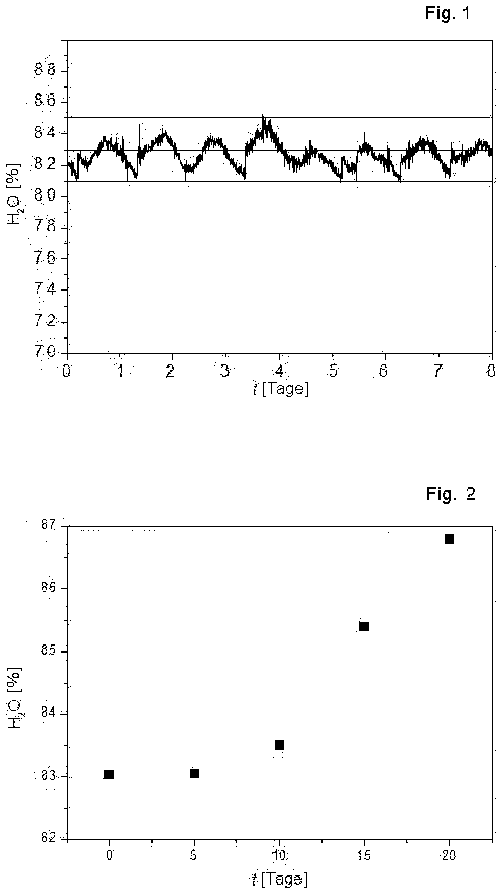

Die schematische Darstellung in Figur 1 zeigt den Wassergehalt des selektiven Lösungsmittels in Gew.-%, bezogen auf das Gesamtgewicht des selektiven Lösungsmittels, H2O, in Gew.-%, in einer Miniplant-Anlage zur Extraktivdestillation eines C4-Schnittes in Abhängigkeit von der Zeit, t, in Tagen. Der Wassergehalt wurde kontinuierlich mittels NIR-Spektroskopie, auf einem NIR-Spektrophotometer der Firma Bruker, Modell Vector 22/N gemessen, und auf einen vorgegebenen Bereich um den Sollwert, 8,3 Gew.-% Wasser, bezogen auf das Gesamtgewicht des selektiven Lösungsmittels, geregelt. Die Darstellung zeigt, dass der Wassergehalt automatisch innerhalb einer Bandbreite von 8,1 Gew.-% als Mindestwert und 8,5 Gew.-% als Maximalwert, geregelt werden konnte.

Die schematische Darstellung in Figur 2 zeigt, dass nach einer klassischen Wasserbestimmung mittels Karl-Fischer-Titration dieselbe Probe, ursprünglich enthaltend 8,3 Gew.-% Wasser, Rest NMP, bezogen auf das Gesamtgewicht des selektiven Lösungsmittels, mit zunehmendem Zeitabstand der jeweiligen Messung falsche Ergebnisse für den Wassergehalt anzeigt.

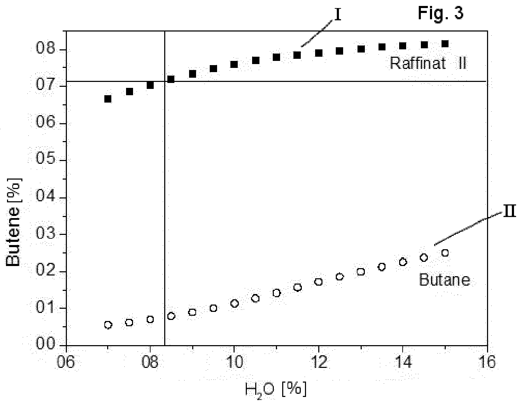

Die schematische Darstellung in Figur 3 zeigt die Abhängigkeit der Produktzusammensetzung vom Wassergehalt des selektiven Lösungsmittels am Beispiel des Anteils an Butenen im Raffinat 2 (obere Kurve I) sowie im Butane enthaltenden Produktstrom (untere Kurve II) einer Extraktivdestillation eines C4-Schnittes zur Butane/Butene-Trennung unter Einsatz eines Gemisches von NMP und Wasser als selektivem Lösungsmittel.

## Patentansprüche

1. Kontinuierliches Verfahren zur Auftrennung eines Gemisches von Kohlenwasserstoffen durch Extraktivdestillation mit einem Gemisch aus einem polaren aprotischen Lösungsmittel und Wasser als selektivem Lösungsmittel, in einer Anlage umfassend eine Extraktivdestillationskolonne und eine Ausgaserkolonne, **dadurch gekennzeichnet, dass** das Verfahren unter kontinuierlicher Messung des Wassergehaltes des selektiven Lösungsmittels durch NIR-Spektroskopie durchgeführt wird, wobei die Messwerte aus der kontinuierlichen Messung des Wassergehaltes des selektiven Lösungsmittels mit einem vorgegebenen Soll-Wert verglichen und durch Ergänzung oder Ausschleusung von Wasser oder durch Ergänzung von polarem aprotischen Lösungsmittel auf einen Wert innerhalb eines vorgegebenen Konzentrationsbereichs oberhalb und unterhalb des Sollwerts geregelt werden.

2. Verfahren nach Anspruch 1, **dadurch gekennzeichnet, dass** die Messwerte aus der kontinuierlichen Messung des Wassergehaltes des selektiven Lösungsmittels zur automatischen Regelung des Wassergehaltes des selektiven Lösungsmittels genutzt werden, indem bei Unterschreiten eines Mindestwertes eine Dosierpumpe in Betrieb gesetzt wird, die Wasser ergänzt oder bei Überschreiten eines Höchstwertes über ein Ventil am Rücklauf der Ausgaserkolonne Wasser ausgeschleust oder über eine Dosierpumpe polares aprotisches Lösungsmittel ergänzt wird.

3. Verfahren nach Anspruch 2, **dadurch gekennzeichnet, dass** die Messwerte aus der kontinuierlichen Messung des Wassergehaltes des selektiven Lösungsmittels zur automatischen Regelung des Wassergehaltes des selektiven Lösungsmittels genutzt werden, indem bei Unterschreiten eines Mindestwertes bei zwei oder mehreren unmittelbar aufeinanderfolgenden Messungen eine Dosierpumpe in Betrieb gesetzt wird, die Wasser ergänzt oder bei Überschreiten eines Höchstwertes bei zwei oder mehreren unmittelbar aufeinanderfolgenden Messungen über ein Ventil am Rücklauf der Ausgaserkolonne Wasser ausgeschleust oder über eine Dosierpumpe polares aprotisches Lösungsmittel ergänzt wird.

4. Verfahren nach einem der Ansprüche 1 bis 3, **dadurch gekennzeichnet, dass** das Gemisch von Kohlenwasserstoffen ein C₄-Schnitt ist.

5. Verfahren nach Anspruch 4, , **dadurch gekennzeichnet, dass** die Extraktivdestillation eine Butane/Butene Trennung ist.

6. Verfahren nach Anspruch 4, **dadurch gekennzeichnet, dass** die Extraktivdestillation des C₄-Schnittes zu 1,3-Butadien als Wertprodukt durchgeführt wird.

7. Verfahren nach einem der Ansprüche 4 bis 6, **dadurch gekennzeichnet, dass** der C₄-Schnitt durch Oxydehydrierung von Butenen gewonnen wurde.

8. Verfahren nach einem der Ansprüche 1 bis 7, **dadurch gekennzeichnet, dass** das polare aprotische Lösungsmittel ausgewählt ist aus N-Methylpyrrolidon, Dimethylformid und Dimethlysulfoxid.

9. Verfahren nach Anspruch 8, **dadurch gekennzeichnet, dass** das polare aprotische Lösungsmittel N-Methylpyrrolidon ist.

10. Verfahren nach Anspruch 9, **dadurch gekennzeichnet, dass** das selektive Lösungsmittel eine Lösung aus Methylpyrrolidon und 7 bis 10 Gew.-% Wasser, bevorzugt aus N-Methylpyrrolidon und 8,3 Gew.-% Wasser, bezogen auf das Gesamtgewicht des selektiven Lösungsmittels, ist.

11. Verfahren nach einem der Ansprüche 1 bis 10, **dadurch gekennzeichnet, dass** die kontinuierliche Messung des Wassergehaltes des selektiven Lösungsmittel durch NIR-Spektroskopie an einer Stelle im Verfahren durchgeführt wird, an der das selektive Lösungsmittel von Kohlenwasserstoffen ausgegast ist.

## Claims

1. A continuous process for separating a mixture of hydrocarbons by extractive distillation using a mixture of a polar aprotic solvent and water as selective solvent in a plant comprising an extractive distillation column and a degassing column, wherein the process is carried out with continuous measurement of the water content of the selective solvent by NIR spectroscopy, with the measured values from the continuous measurement of the water content of the selective solvent being compared with a prescribed intended value and being regulated to a value within a prescribed concentration range above and below the intended value by introduction or removal of water or by introduction of polar aprotic solvent.

2. The process according to claim 1, wherein the measured values from the continuous measurement of the water content of the selective solvent are utilized for automatic regulation of the water content of the selective solvent by actuating a metering pump which introduces water when the measured value falls below a minimum value or discharging water via a valve on the runback conduit of the degassing column or introducing polar aprotic solvent via a metering pump when a maximum value is exceeded.

3. The process according to claim 2, wherein the measured values from the continuous measurement of the water content of the selective solvent are utilized for automatic regulation of the water content of the selective solvent by actuating a metering pump which introduces water when two or more immediately successive measurements are below a minimum value or water being discharged via a valve on the runback conduit of the degassing column or polar aprotic solvent being introduced via a metering pump when two or more immediately successive measurements exceed a maximum value.

4. The process according to any of claims 1 to 3, wherein the mixture of hydrocarbons is a C₄ fraction.

5. The process according to claim 4, wherein the extractive distillation is a separation of butanes and butenes.

6. The process according to claim 4, wherein the extractive distillation of the C₄ fraction is carried out to give 1,3-butadiene as desired product.

7. The process according to any of claims 4 to 6, wherein the C₄ fraction has been obtained by oxydehydrogenation of butenes.

8. The process according to any of claims 1 to 7, wherein the polar aprotic solvent is selected from among N-methylpyrrolidone, dimethylformamide and dimethyl sulfoxide.

9. The process according to claim 8, wherein the polar aprotic solvent is N-methylpyrrolidone.

10. The process according to claim 9, wherein the selective solvent is a solution composed of methylpyrrolidone and from 7 to 10% by weight of water, preferably N-methylpyrrolidone and 8.3% by weight of water, based on the total weight of the selective solvent.

11. The process according to any of claims 1 to 10, wherein the continuous measurement of the water content of the selective solvent by NIR spectroscopy is carried out at a point in the process at which the selective solvent has been freed of hydrocarbons by degassing.

## Revendications

1. Procédé continu pour la séparation d'un mélange d'hydrocarbures par distillation par extraction avec un mélange constitué par un solvant aprotique polaire et par de l'eau comme solvant sélectif, dans une installation comprenant une colonne de distillation par extraction et une colonne de dégazage, **caractérisé en ce que** le procédé est réalisé sous une mesure continue de la teneur en eau du solvant sélectif par spectroscopie NIR (Near Infrared - proche infrarouge), les valeurs de la mesure continue de la teneur en eau du solvant sélectif étant comparées à une valeur de consigne prédéfinie et régulées par un complément ou un soutirage d'eau ou par un complément de solvant aprotique polaire à une valeur dans une plage de concentration prédéfinie au-dessus et sous la valeur de consigne.

2. Procédé selon la revendication 1, **caractérisé en ce que** les valeurs de la mesure continue de la teneur en eau du solvant sélectif pour la régulation automatique de la teneur en eau du solvant sélectif sont utilisées **en ce que**, lors du passage sous une valeur minimale, une pompe de dosage qui complète l'eau est démarrée ou, lors du passage au-dessus d'une valeur maximale, de l'eau est soutirée via une soupape au niveau du reflux de la colonne de dégazage ou le solvant aprotique polaire est complété via une pompe de dosage.

3. Procédé selon la revendication 2, **caractérisé en ce que** les valeurs de la mesure continue de la teneur en eau du solvant sélectif pour la régulation automatique de la teneur en eau du solvant sélectif sont utilisées **en ce que**, lors du passage sous une valeur minimale lors de deux mesures, ou plus, directement consécutives, une pompe de dosage qui complète l'eau est démarrée ou, lors du passage au-dessus d'une valeur maximale lors de deux mesures, ou plus, directement consécutives, de l'eau est soutirée via une soupape au niveau du reflux de la colonne de dégazage ou le solvant aprotique polaire est complété via une pompe de dosage.

4. Procédé selon l'une quelconque des revendications 1 à 3, **caractérisé en ce que** le mélange d'hydrocarbures est une fraction en C₄.

5. Procédé selon la revendication 4, **caractérisé en ce que** la distillation par extraction est une séparation butanes/butènes.

6. Procédé selon la revendication 4, **caractérisé en ce que** la distillation par extraction de la fraction en C₄ est réalisée vers le 1,3-butadiène comme produit valorisable.

7. Procédé selon l'une quelconque des revendications 4 à 6, **caractérisé en ce que** la fraction en C₄ est obtenue par oxydéshydrogénation de butènes.

8. Procédé selon l'une quelconque des revendications 1 à 7, **caractérisé en ce que** le solvant aprotique polaire est choisi parmi la N-méthylpyrrolidone, le diméthylformamide et le diméthylsulfoxyde.

9. Procédé selon la revendication 8, **caractérisé en ce que** le solvant aprotique polaire est la N-méthylpyrrolidone.

10. Procédé selon la revendication 9, **caractérisé en ce que** le solvant sélectif est une solution de méthylpyrrolidone et de 7 à 10% en poids d'eau, de préférence de N-méthylpyrrolidone et de 8,3 % en poids d'eau, par rapport au poids total du solvant sélectif.

11. Procédé selon l'une quelconque des revendications 1 à 10, **caractérisé en ce que** la mesure continue de la teneur en eau du solvant sélectif par spectroscopie NIR est réalisée en un endroit dans le procédé où le solvant sélectif est dégazé des hydrocarbures.
